Europäisches Patentamt

(19) European Patent Office

Office européen des brevets

(11) **EP 0 911 401 A1**

(12) **EUROPEAN PATENT APPLICATION**

(43) Date of publication:
28.04.1999 Bulletin 1999/17

(51) Int. Cl.$^6$: **C12N 15/24**, C12N 1/21,
C07K 14/54

(21) Application number: 97118219.1

(22) Date of filing: 21.10.1997

| | |
|---|---|
| (84) Designated Contracting States:<br>AT BE CH DE DK ES FI FR GB GR IE IT LI LU MC<br>NL PT SE<br>Designated Extension States:<br>AL LT LV RO SI | (71) Applicant: **BAYER AG**<br>**51368 Leverkusen (DE)**<br><br>(72) Inventor:<br>**Sebald, Walter, Prof. Dr.**<br>**97074 Würzburg (DE)** |

(54) **Muteins of interleukin 4 showing low-affinity and short-term interaction with the common gamma chain**

(57)     Human IL-4 (IL-4), one of the small 4-helix-bundle cytokines, uses the specific IL-4 receptor a chain together with a promiscuous subunit, the common $\gamma$ chain ($\gamma_c$) for transmembrane signaling. The ligand-binding properties of $\gamma_c$, which are presently poorly understood, were analysed by biosensor techniques employing recombinant ectodomains gamex ($\gamma_c$) and IL4-BP (a) of the receptor chains. The formation and decay of a ternary complex between solute gamex and IL-4 liganded IL4-BP could be established to exhibit a low affinity ($K_d = 3\mu M$) as well as a short half life $t_{1/2} = 7s$. This binding affinity resulted to the major part from the interaction of gamex with IL-4 and not from a direct contact of IL4-BP and gamex, since the binary complex between solute gamex and immobilized IL-4 showed an only 50fold greater $K_d$ of 150 µM. The IL-4 residues involved in gamex binding were identified by means of an alanine-scanning mutational approach. A functional gamex binding IL-4 epitope is proposed comprising I11, N15, and Y124 as major determinants. Even IL-4 variants which bind gamex 300fold weaker than IL-4 with a dissociation half life $t_{1/2}$ of less than 1s, retained a substantial T-cell proliferative activity. These findings suggest that low affinity $\gamma_c$ binding and short half lives of the heterodimeric a/$\gamma_c$ receptor complex are sufficient for initiating IL-4 dependent signal transduction.

## Description

Hematopoietic cytokines generate a transmembrane signal by promoting the dimerisation of two receptor chains (see Heldin, 1995). The small helical cytokines (see Rozwarski et al., 1994) usually employ receptors with two different receptor chains and upon ligation a heterodimer is formed. One of the receptor chains determines the specificity in cytokine binding by providing a high- or intermediate-affinity binding site. The second chain often has no measurable intrinsic affinity itself but in certain systems can confer high affinity binding to the receptor heterodimer. The second chain is usually promiscuous and serves as a common chain in different receptor systems as in the $\beta_c$ or $\gamma_c$ families of cytokine receptors (see Sugamura et al., 1995). The affinity, kinetics and specificity of common chain binding are poorly understood.

The heterodimeric IL-4 receptor system (see Duschl and Sebald, 1996) contains an a chain which determines the specific high-affinity binding of the IL-4 ligand (Kruse et al., 1993). It uses $\gamma_c$ as the second chain in common with the receptor systems for IL-2, IL-7, IL-9, and IL-15 (see Sugamura et al., 1995; Leonard, 1994). An inherited loss in the common $\gamma$ chain leads to a severe combined immunodeficiency (XSCID) both in humans or mice in accordance with the essential function of $\gamma_c$ in the signaling of several cytokines. Monoclonal antibodies to $\gamma_c$ block the activity of IL-4 as well as that of the other cytokines of the $\gamma_c$ family (see Sugamura et al., 1996). The signaling capacity of the IL-4 receptor is located mainly on the large cytoplasmic part of the a chain. The short 86-residue cytoplasmic tail of $\gamma_c$ has been demonstrated to anchor the Jak 3 tyrosine kinase (see Ihle et al., 1995), which together with $\gamma_c$ is essential for IL-4 dependent signaling.

Neither IL-4 nor any other cytokine up to now could be demonstrated to bind directly to $\gamma_c$ in the absence of the a chain (see Sugamura et al., 1995). The significance of this observation is uncertain, however, since in whole cells containing both the a and the $\gamma_c$ chain a physical interaction of IL-4 and $\gamma_c$ could be established by chemical cross-linking experiments. Furthermore, antagonistic IL-4 variants mutated at certain residues on helix D. (Duschl, 1995) were no longer cross-linked with $\gamma_c$, supporting the previous conclusion that these antagonistic variants map at an IL-4 site interacting with the second receptor chain $\gamma_c$ ( Kruse et al., 1993). An IL-4 dependent association between $\gamma_c$ and the a chain was indicated by immunoprecipitation experiments employing $\gamma_c$ specific monoclonal antibodies. Originally, the IL-2 dependent association of $\gamma_c$ with the Il-2 receptor a and b chains has been exploited by Sugamura and cow. for the identification and cloning of $\gamma_c$ Takeshita et al., 1992;). Direct evidence for the formation of an complex between IL-4 and the extracellular binding domains of the a and $\gamma_c$ chain was obtained by gel filtration experiments employing the recombinant soluble receptor domains (Hoffman et al., 1997). A ternary complex with a 1:1:1 stoichiometry between IL-4, IL4-BP and gamex was reported. A rough estimate yielded a $K_d$ in the μM range for the interaction between gamex and the IL-4/IL-4-BP complex.

In the IL-4 receptor system the $\gamma_c$ chain has only a marginal effect on the IL-4 binding affinity to the whole complex. Cells expressing a functional IL-4 receptor with both the a and the $\gamma_c$ chain exhibited an only 3 to 5fold higher affinity for IL-4 compared to cells expressing the a chain alone. A similarly small contribution of $\gamma_c$ to ligand binding in whole cells has been established for the heterodimeric IL-7 and IL-9 receptor systems (see Sugamura et al., 1995). A complicated pattern emerged from binding studies with the heterotrimeric IL-2 receptor system. The human IL-2 receptor $\beta$ or $\gamma_c$ chain alone showed no affinity ( Kd > 0,1 - 1 μM ) for the IL-2 ligand. The $\beta$ chain increased, however, the IL-2 affinity of the a chain from a $K_d$ of 10 nM to a $K_d$ of 100 pM (100fold), and the $\gamma_c$ chain increased the IL-2 affinity of an Il-2 receptor a / $\beta$ complex in fibroblastoid cells a further 10fold (see Sugamura et al., 1995).

The specific high-affinity interaction between IL-4 and the extracellular part of the a chain (IL4-BP) has a $K_d$ of 150 pM ( Kruse et al., 1993; Shen et al., 1996) This binary complex associates rapidly ($k_{on} = 1,5 \times 10^7 \, M^{-1} \, s^{-1}$), because its formation is facilitated by electrostatic steering. The IL-4/IL4-BP complex has a long half life ($t_{1/2}$ = 350 sec). This is in the same time range as the internalisation of the receptor-bound IL-4 in whole cells. On the IL-4 side this interaction is determined by a functional epitope constituted of a mixed charge pair (E9, R88), three polar (T13, R53, N89) and two hydrophobic (I5, W91) amino acid side chains located on helix A and C ( Wang et al., 1997).

Residues of the $\gamma_c$-binding epitope were tentatively located on IL-4 helix D. Substitutions of R121, Y124, and S125 by aspartate yielded IL-4 variants with partial agonist/antagonist activity, while retaining high-affinity binding to the a chain ( Kruse et al., 1992; Kruse et al., 1993). A perfect antagonist was created by introducing two aspartate residues at positions 121 and 124 ( Tony et al., 1994). The antagonistic effect of these substitutions, however, seemed to result more from a mismatch with $\gamma_c$ rather then from the loss of binding side chains (Müller et al., 1994). Thus, the identity of the $\gamma_c$ -binding residues of IL-4 remained uncertain.

A sequential 2-step mechanism has been proposed for the activation of the IL-4 receptor (Kruse et al., 1993) and for several other cytokine receptor systems (see Wells, 1994), in order to accommodate the different affinities of the cytokine for the two receptor chains and to explain the existence of high-affinity antagonists. As shown in the present communication, IL-4 has an at least $10^4$-fold lower affinity for the extra-cellular part of $\gamma_c$ (gamex) than for that of the a chain (IL4-BP). This offered the unique experimental possibility to assemble stepwise the ternary receptor complex on a biosensor matrix, and to analyse the physical binding of gamex to a preformed IL-4/IL4-BP complex. In this way the energetics and the kinetics of this interaction could be determined. Employing a collection of IL-4 variants carrying alanine substitutions of residues at helices D and A, the contributions of individual side chains to $\gamma_c$-binding, i.e. the functional epitope for $\gamma_c$ interaction, could be determined. The physical binding data were compared with the biological activity of alanine variants affected in the functional binding epitope.

The present results support the view that a stable IL-4/a chain complex becomes activated and signaling is induced by a short-term and low-affinity interaction with the common $\gamma_c$ chain. Because low-affinity interactions as well as a 2-step activation mechanism have been described for other cytokine and growth factor receptor systems (see e.g. Wells, 1994) a mechanism governed by high and low affinity interactions with two receptor chains might apply to them as well. The identification of the functional residues in site 2 of human Il-4 provides the basis for the construction of further IL-4 antagonists.

1. Formation of a ternary complex between gamex and IL-4/IL4-BP

IL-4 induces the formation of a heterodimeric receptor in two steps. First, site 1 of IL-4 associates with IL4-BP, the extracellular domain of the receptor a chain. In a second step gamex, the extracellular domain of the common $\gamma$ chain, binds to the IL-4/IL4-BP complex. Site 2 of IL-4 is involved in this interaction. The formation and decay of the ternary complex by association and dissociation of gamex can be analysed on a biosensor in real time.

The recombinant IL4-BP had been immobilized at a streptavidin-coated matrix by means of a C-terminal biotin label. Perfusion with high concentration of IL-4 saturated the binding protein. When IL-4 perfusion was continued together with a recombinant extracellular domain of $\gamma_c$ (gamex), a concentration-dependent association of gamex was observed.The bound gamex dissociated rapidly when the complex was perfused with buffer alone. The dissociation of IL-4 was slow under these conditions, as determined in parallel experiments in the absence of gamex. The binding of gamex to the IL-4/IL4-BP complex was specific, since a flow cell with streptavidin-coated matrix alone, which was perfused in series, did not measurably interact with gamex under the applied conditions.

From the kinetically controlled phase of gamex association a rate constant $k_{on} = 3,2 \times 10^4 \text{ M}^{-1} \text{ s}^{-1}$ was calculated on the basis of a 1:1 interaction model (Table 1, bottom line). The exponential decay of the complex had a rate constant $k_{off} = 0,11 \text{ s}^{-1}$. From these data, a dissociation constant $K_d = 3,4 \text{ }\mu\text{M}$ was calculated. A similar $K_d$ of 2,8 $\mu$M was obtained when the equilibrium binding at different gamex concentrations was evaluated (Table 2). The highest concentration of gamex applied during these experiments was 30 $\mu$M, corresponding to 1 mg protein per ml. At higher concentrations unspecific binding to the streptavidin matrix increasingly resulted in low signal to noise ratios.

The established kinetic and equilibrium constants for gamex interaction in the ternary complex differed largely from the corresponding constants of the interaction between IL-4 and IL4-BP ($K_d = 1,5 \times 10^{-10}$ M). A half life $t_{1/2} = 8$ s for the dissociation of gamex contrasts with a $t_{1/2} = 400$ s for the decay of the IL-4/IL4-BP complex. The association rate constant for gamex was nearly three orders of magnitude lower than that for IL4-BP and IL-4 ($k_{on} = 1,5 \times 10^7 \text{ M}^{-1} \text{ s}^{-1}$).

Gamex binding to IL-4/IL4-BP was remarkably insensitive to changes in ionic strength, pH and temperature. It was not measurably altered when salt concentration was varied between 50 mM and 1 M NaCl. Kinetics and equilibrium binding were similar at pH values between 6,2 and 8,5. Temperature changes from 6 °C to 37 °C had no influence on gamex interaction with IL-4/IL4-BP (data not shown).

2. Formation of a binary complex between gamex and IL-4.

Gamex did not measurably bind to immobilised IL4-BP in the absence of IL-4. Even at 30 $\mu$M gamex concentration in the perfusate, background levels of interaction were observed, i.e. less than 1% of the binding in the presence of IL-4. It can be therefore estimated, that if this binary interaction exists at all the dissociation constant $K_d$ would be larger than 3 mM.

For the analysis of the interaction of gamex with IL-4, a variant IL-4/cys was constructed allowing the immobilisation of IL-4 via a short C-terminal extension containing a single cysteine (see Methods). Gamex bound to IL-4/cys in the absence of IL4-BP. Scatchard analysis of equilibrium binding yielded for this binary interaction a $K_d$ of 150 μM corresponding to a free energy of binding dG = 5,2 kcal mol$^{-1}$. The kinetic constants could not be evaluated since the binding levels were low in relation to the bulk effects blurring the kinetic phases of the sensograms.

When the immobilized IL-4/cys was first saturated with IL4-BP and the resultant complex was then perfused with gamex (in the presence of IL4-BP) an equilibrium binding with a $K_d$ of 4+/- 1 μM was observed. This is the same value as that obtained during the experiments described in the previous paragraph were immobilized IL4-BP was employed. The affinity of gamex for IL-4 alone was accordingly about 40fold weaker than that for the IL-4/IL4-BP complex. This ratio corresponds to a difference in standard free binding energy ddG = 2,2 kcal mol$^{-1}$. The result of this calculation strongly suggests that the main part of gamex binding affinity in the ternary complex originated from the interaction with IL-4 (dG = 5,2 kcal mol$^{-1}$).

3. <u>The gamex binding epitope of IL-4</u>

The contribution of individual amino acid side chains to gamex binding was studied by an alanine scanning mutational approach. Surface residues on IL-4 helices D and A were singly substituted by alanine and the gamex binding properties of the resulting alanine variants were analysed by the biosensor techniques described above. All IL-4 variants analysed in this study (see Tables 1 and 2) exhibited a largely unaltered binding to IL4-BP.

The measured kinetic constants for interaction of gamex with the complex between IL4-BP and the individual IL-4 variants are compiled in Table 1. In the presence of all IL-4 variants similar association rates were observed. The $k_{on}$ values varied between 1,7 and 3,8 x 10$^{+4}$ M$^{-1}$ s$^{-1}$. Most of the variants showed also unaltered dissociation rate constants, $k_{off}$, similar to that of the wild type IL-4. An only 2 to 3-fold faster $k_{off}$ occurred in alanine variants affected at positions R121, E122, and S125. Five variants, however, exhibited a more than 4-fold increase in the rate constant for the dissociation of gamex from the ternary complex. Residue Y124 has been postulated before to be a main determinant for gamex binding (Kruse et al., 1992). Now in addition contributions of L7, I11, N15 and K123 to this interaction were detected. The $k_{off}$ for IL-4 variant N15A could be evaluated, whereas the $k_{off}$ values for IL-4 variants L7A, I11A and Y124A were larger than 0,5 s$^{-1}$. The latter could not be reliably determined, since the resolution is limited by the data collection rate of the BIA2000 system (2,5 Hz). Variant K123A showed the strongest defect. It was unable to promote gamex binding even at the highest gamex concentration of 30μM.

The dissociation constants $K_d$ for the dissociation of gamex from the ternary complex were evaluated from the concentration dependence of the equilibrium binding. The $K_d$ values were between 15 and 220 μM when IL-4 variants N15A, L7A, I11A, and Y124A were employed (Table 2). The $K_d$ of IL-4 variant K123A was too high to allow gamex binding up to a concentration of 30 μM. Thus, only a lower limit of about 300 μM can be estimated for the $K_d$ of this variant. The $K_d$ values of several other alanine variants mutated at Il-4 helix D (E114A, K117A, T118A, R121A, E122A, and S125A) were found to be increased two- to threefold compared to the $K_d$ of IL-4. An only twofold increase in $K_d$ occurred also in variant K12S.

In summary, a defined value for the kinetic constants, $k_{on}$ and $k_{off}$, as well as for the dissociation constant, $K_d$, could be determined only for a group of IL-4 variants with small alterations in gamex binding, as e.g. N15A. For a second group of IL-4 variants, i.e. L7A and I11A, the $k_{on}$ could be experimentally analysed, but not the $k_{off}$. Because the $K_d$ of these variants could be determined by Scatchard analysis (equilibrium binding), the $k_{off}$ could be still computed as $K_d * k_{on}$. The calculated dissociation rate constants $k_{off}$ for these IL-4 variants are 0,7 s$^{-1}$ (L7A) and 1,7 s$^{-1}$ (I11A). For the third group of IL-4 variants, i.e. for Y124A and K123A, neither $k_{on}$ nor $k_{off}$ could be directly measured. Only the dissociation constant $K_d$ could be determined for IL-4 variant Y124A and a lower limit could be given for the $K_d$ of K123A. Based on the assumption that the association rate constants $k_{on}$ for Y124A and K123A are similar as those of the other variants and of IL-4, the dissociation rate constants $k_{off}$ can be estimated to be 7 s$^{-1}$ (Y124A) and >10 s$^{-1}$ (K123A). The corresponding half lifes $t_{1/2}$ of the ternary complexes between the IL-4 variants, IL4-BP and gamex would be 1 s (L7A), 400 ms (I11A), 100 ms (Y124A) and <70 ms (K123A).

The mutational analyses did not discriminate between structural and binding effects of an alanine substitution. The unaltered IL4-BP affinity as well as the similar behaviour during refolding and purification suggested that no gross structural perturbations had been produced in any of the IL-4 mutant proteins. Nevertheless, in two of the variants, namely L7A and K123A, the reduced affinity for gamex probably originated from structural effects of the alanine substitution. The residue L7 exposes only 0,4 Å$^2$ at the surface of the IL-4 molecule. In all established structures of IL-4 residue L7 has been established as part of the hydrophobic core (see e.g. Müller et al., 1994a). The small accessible surface of K123 (12,5 $\text{Å}^2$) is oriented away from the helix AD face of IL-4. Remarkably, substitution of L7 and K123 by the negatively charged aspartyl residue produced no major loss in affinity, in contrast to the positions of I11 and Y124 ( and also those of N15, R121 and S125 ) were aspartate substitutions lead to a much higher loss in affinity than the alanine substitutions ( see Table 2).

In the 3-dimensional structure of IL-4 the other probably functional binding determinants represent a contiguous patch clustered on the surfaces of helices D and A. Two of the main binding residues, namely Y124 and I11, have large accessible surface areas of 27,2 and 17,3 $\text{Å}^2$, respectively. Residues N15 together with K12 and surface residues on helix D (with the exception of R115) form a semicircle of weaker binding determinants. The loss of binding free energy due to alanine substitution calculated as ddG = 1,36 log $K_d$(variant)/ $K_d$(IL-4) was 2,6 kcal/mol (Y124A), 2 kcal/mol (I11A), and 1 kcal/mol (N15A) for the three main binding residues.

The proposed $\gamma_c$ binding epitope of IL-4 is largely hydrophobic. Even the positions of R121, S125, and Y124 can be substituted by large hydrophobic side chains without great effect on gamex binding. IL-4 variants studied comprise R121W, R121L, Y124F, S125W, and S125I.

4. Biological activity

Remarkably, the loss in gamex binding affinity as compiled in Table 2, was not linearly related to the loss in biological activity during a T-cell proliferation assay. This applies both for the alanine and the charge variants. Variants [K123A]IL-4 and [Y124A]IL-4, which were both dramatically impaired in gamex binding still produced 16% or 50%, respectively of the maximal biological response of IL-4. The small loss in potency of [L7A]IL-4 was at the limit of detection. The variants N15A, E114A, R121A, E122A, and S125A, which had noteabel defects in gamex binding, were unconspicuous when tested in the T-cell proliferation assay. With all alanine variants analysed the $EC_{50}$, the concentration resulting in halfmaximal response, was similar to the $EC_{50}$ = 120 pM of IL-4. The charge variants of IL-4 compiled in Table 2 showed the same tendency as the alanine variants. An up to 100fold reduced gamex binding affinity (see e.g. N15D and T118K) was correlated with a marginally decreased maximal proliferative response. Variants I11D, R121D, and S125D exhibited a more than 300fold lower binding affinity and still produced 15 to 20% of the maximal IL-4 response. IL-4 variant Y124D represented a special case, since it exerted no measurable proliferative activity in T-cells, as has been described before (Kruse et al., 1992).

The unaltered IL4-BP binding-affinity and $EC_{50}$ values, the partial agonist activities of variants I11A, K123A, and Y124A corresponded to their partial antagonist activities for T-cell proliferation. The maximal inhibition of IL-4 stimulated T-cell proliferation was between 84% (K123A) and 35% (I11A).

In conclusion, these results indicated that a more than 100-fold reduced binding affinity of IL-4 for gamex still allows a pronounced biological activity during T-cell proliferation. The half-lives of ternary complex formed between IL4-BP, gamex and some of the investigated IL-4 variants were below $t_{1/2}$ < 1 sec. Such short times seem to be sufficient for receptor activation.

Interleukin 4, one of the small helical cytokines (for definition see Rozwarski et al., 1994), activates its cognate receptor by a sequential binding of two different receptor chains (heterodimer formation). The functional epitope at site 1 of human IL-4 which binds the receptor a chain has been described before (Wang et al., 1997). Now site 2 interacting with the common $\gamma_c$ chain was characterised in functional and structural detail. The interaction at site 2 of IL-4 was found to differ fundamentally from that at site 1. This applies (1) for the type of the functional binding residues of IL-4, (2) the affinity and specificity of the binding, and (3) the kinetics of the interaction.

IL-4 site 2 is hydrophobic and permissive. Two hydrophobic residues (I11, Y124) at site 2 of IL-4 represent the main determinants for $\gamma_c$ binding. Large hydrophobic residues substituted at positions of functional residues R121, Y124 and S125 dont lead to a loss in $\gamma_c$ binding. Accordingly, IL-4 site 2 tolerates different amino acid side chains as long as an overall hydrophobicity is retained. Such a permissive side chain usage probably reflects the promiscuous interaction of the complementary binding site on the $\gamma_c$ chain with the corresponding site 2 of IL-2, IL-7, IL-9 and IL-15. The similarities of the IL-4 site 2 with the postulated $\gamma_c$ binding site of IL-2 are conspicuous Rozwarski et al., 1994, Müller et al., 1994a). Possibly, the permissive side chain usage in site 2 is also related to the observation, that human IL-4 is able to functionally interact with murine $\gamma_c$. It probably can bind still another receptor protein, the IL- 13 Ra1 chain, which functions as a second chain (g') during IL-4/a chain signalling (Aman et al., 1996; Miloux et al., 1996; Gauchat et al., 1997). The IL-4 site 1 epitope for a chain binding, in contrast, is highly species-specific. It has been localised at helices A and C and found to be composed predominantly of polar or charged residues (Wang et al., 1997).

"A hot spot of hydrophobic binding energy" in IL-4 site 2. The accumulated loss in free binding energy for all functional alanine variants in site 2, not counting L7A and K123A, amounts to 10 kcal mol$^{-1}$. The three main determinants together, i.e. I11 N15 and Y124, account for 5,6 kcal mol$^{-1}$, i.e. more than half of the total value. The rest is equally distributed among seven other side chains predominantly at helix D. As a common theme, both in site 2 and site 1 of IL-4, the receptor binding affinity seems to be concentrated in a few key residues and some surrounding satellite side chains. However, the postulated binding increments of the individual functional side chains in site 2 of IL-4 were nearly an order of magnitude lower than those at site 1. This supports the notion that the IL-4/$\gamma_c$ interaction has a low affinity (see below). The site 1 interaction in the related growth hormone receptor system also has been found to be concentrated in a few key side chains (K172, T175, F176, and R178) at helix D which can engage in hydrophobic interactions (Cunningham and Wells, 1993;). The sum of the reductions in free binding energies caused by alanine or glutamine substitutions at the growth hormone site 1, however was about 15 kcal mol$^{-1}$ as to expect for a high-affinity binding.

Relevance of the biosensor interaction analysis for the dimerisation of receptor chains in the membrane. During the present biosensor experiments solute gamex , a soluble extracellular domain of $\gamma_c$, reacted with an immobilized IL-4/IL4-BP complex. This 3-dimensional situation has to be compared with a restricted 2-dimensional diffusion of complete receptor chains in the plane of the plasma membrane. The assembly of $\gamma_c$ with the IL-4 loaded a chain in the membrane can be assessed only indirectly, as discussed further below. From theoretical considerations it has been concluded that the preorientation of membrane proteins, i.e. a restricted rotational diffusion, can enhance the probability of a productive collision and therefore the association rate constant by several orders of magnitude. The slower diffusion rates of proteins in the membrane compared to that in free solution, however, would reduce the association rate. Thus, it remains uncertain in how far the gamex association rate $k_{on}$ of 3 x 10$^{+4}$ M$^{-1}$ s$^{-1}$ determined during the biosensor studies deviates from the association rate in the membrane.

Anyhow, the dissociation rate constant $k_{off}$ for the decay of the ternary complex seems to be more important for $\gamma_c$ binding than the $k_{on}$, since after alanine or charged residue substitution at IL-4 site 2 predominantly the dissociation rate for gamex was accellerated, wheras the the $k_{on}$ remained more or less the same in the presence of all analysed variants. The dissociation rate constant in a first approximation is independent of the protein concentration and of diffusion rates. Thus, it may be concluded that dissociation of gamex from IL-4/IL4-BP at the biosensor has a similar rate constant as the dissociation of $\gamma_c$ from IL-4/a chain in the membrane.

Ligand-affinity conversion by $\gamma_c$. In the IL-4 receptor system the affinity for gamex binding was determined to the most part by IL-4. The affinity of gamex for the "empty" IL4-BP was exceedingly low as deduced from the only 50fold difference in the affinity of gamex for IL-4 or the IL-4/IL4-BP complex, respectively. Such a weak interchain affinity is in accordance with the finding that the affinity of IL-4 for the receptor a chain in whole cells is increased only 3 to 5fold by the addition of the $\gamma_c$ chain (see Sugamura et al., 1995). In other cytokine receptor systems, especially in the common $\beta$ chain ($\beta_c$) family of receptors (see below), the increase in ligand affinity due to the second chain was found to be larger, and this effect has been termed affinity conversion. Affinity conversion due to conformational effects has not been disproven. It is obvious, however, that a substantial affinity conversion can occur when binding affinity between the second and the first receptor chain exists, in addition to the affinity of the second chain for the cytokine ligand. In receptors of the $\gamma_c$ family, as in the IL-7 and IL-9 receptors, affinity conversion is about 5fold, similar as in the IL-4 receptor (see Sugamura et al., 1995). The affinity of the IL-2 receptor a/b chain complex for IL-2 is increased about 10fold after addition of the $\gamma_c$ chain. Accordingly, the $\gamma_c$ chain has a very low ligand-independent affinity for the specific chain in all these receptors. For cytokines IL-3, IL-5, and GM-CSF, which use the common $\beta$ chain ($\beta_c$) as second chain for signalling, no binding to $\beta_c$ could be measured up to now, probably since the high ligand concentration necessary for the binding at µM dissociation constants had not been attained. A 1000fold affinity conversion of the a chain was observed, however, for IL-3 in the presence of $\beta_c$. The increase for GM-CSF was found to be 100fold. A 3 to 5fold affinity conversion of the a chain was observed with IL-5. The latter increase is comparable to the IL-4 system. It appears possible, that different affinities of $\beta_c$ for the various a chains produce these different affinity conversions.

Receptor signaling in the absence of a ligand. In principle, higher interchain affinities can be expected to increase the basal signaling activity of a receptor system in the absence of a ligand. A ligand specific activation via heterodimerisation requires only direct interactions between the cytokine ligand and the ectodomains of the first and second receptor chain. A basal receptor activity might be counterbalanced by a deactivation reaction, e.g. by a protein-phosphatase acting on a cytokine receptor. Such a dynamic situation might allow a more rapid adaptation to specific signalling requirements.

The transmembrane signaling of the IL-4 receptor can be initiated by a short-term dimerisation of the a and $\gamma_c$ chains. The alanine substitutions of the functional residues in IL-4 site 2 have a remarkably little effect on biological activity Even IL-4 variant K123A, which shows the largest loss in gamex binding, still has a 16% partial agonist activity and an unchanged EC$_{50}$ for T-cell proliferation. Because activated T-cells are quite insensitive to IL-4 (EC$_{50}$ 200 pM), the K123A variant probably would be unconspicuous in the much more sensitive B-cell assay for CD23 induction (EC$_{50}$

ca 10 pM) (see Kruse et al., 1992). IL-4 Variant Y124A exhibiting a 80fold reduced gamex binding affinity retained 50% partial agonist activity in T-cells. This suggests that a short-lived IL-4-triggered contact between the ectodomains of the receptor a and $\gamma_c$ chain with a half-life in the 100 ms range can initiate receptor transmembrane signaling. In line with this conclusion are the previous findings that the construction of a perfect antagonistic IL-4 variant required the incorporation of two aspartyl residues at site 2 (Tony et al., 1994). Apparently, it is not sufficient to introduce loss-of-binding, i.e. alanine substitutions at this site to abolish receptor activation. A mismatch which causes a repulsion between IL-4 and gamex appears to be necessary to prevent activation (Müller et al., 1994a). It is interesting to note, that a mismatch at position 124 in IL-4 variant Y124D has a large disruptive effect for the interaction with human $\gamma_c$, whereas the disruptive effect is small for murine $\gamma_c$ and IL-13Ral. The reverse is true for a mismatch at position 121 in variant R121D (Schnarr et al., 1997). Accordingly, these variants behave as selective agonists. which discriminate the different IL-4 receptor systems.

Short-lived low-affinity interactions in transmembrane signaling. The physiological relevance of a short-term interaction with the $\gamma_c$ chain remains uncertain. It could simply indicate that the heterodimerisation step is not rate-limiting for the transactivation by Jak3 or other tyrosine kinases. It is probably worthwhile to consider the possibility, however, that a serial engagement of $\gamma_c$ with many ligand-occupied a chains could mean a gain in sensitivity, similar to the T-cell receptor (TCR) system. A few peptide-loaded MHC complexes by serial engagement can activate many TCR complexes ("high-sensitivity/low-affinity paradox"; see Valltutti et al., 1995). The two sites at the MHC I/peptide complex engage in low-affinity short-term interactions with TCR and CD8, respectively (Garcia et al., 1996). Furthermore, the MHC-mediated heterodimerisation of TCR and CD8 seems to initiate specific lck-dependent signaling events in the receptor. Concerning the IL-2, -4, -7, -9, -15 receptor systems it is presently undetermined if the $\gamma_c$ chain is present in substoichiometric amounts compared to the a or a/b chains, or if $\gamma_c$ might be limiting in certain cells at certain developmental or differentiation stages.

The low-affinity interaction observed with the recombinant soluble ectodomains seems to be at variance with the finding that $\gamma_c$ can form quite stable aggregates with the IL-4 receptor a chain, as deduced from immunoprecipitation experiments in BaF3-derived cell lines, where $\gamma_c$ has been recovered with the a chain by means of anti $\gamma_c$ chain antibodies. Similarly, $\gamma_c$ was originally isolated and identified by coprecipitation with the IL-2 receptor $\beta$ chain (Takeshita et al., 1992). It can be visualized, however, that in the membrane the primary event after cytokine binding is the formation of a short-term low affinity heterodimer between the receptor chains. After some time the heterodimer may be stabilized (maturated), e.g. by the associating signaling proteins in the cytosolic domains (Jaks, STATs, phosphatases, etc.) or by other still unknown mechanisms which might involve the association of transmembrane segments, disulfide-bridge formation between receptor chains, or the cytoskeleton. A receptor heterodimer maturation into a "functional super complex" has been invoked previously (see Darnell et al., 1994) to account for the loss of high-affinity interferon a binding in cell lines with defective or deficient Tyk2 or Jak 1.

## Preparation of receptor domains and IL-4 variants

The [C182A,Q207C]IL4-BP variant was produced in SF9 cells, purified, and biotinylated at Cys207 as described (Shen et al., 1996).

The extracellular domain of the $\gamma_c$ chain (gamex) comprising amino acid residues 23 - 254 (Takeshita et al., 1992) plus an N-terminal extension ADLGSRAMG was produced in SF9 cells employing the pAcGP67B based Baculogold system (Pharmingen). The secreted protein was purified from a serum-free (Insect-Xpress medium Bio*Whittaker) culture supernatant by means of the following chromatographic steps:

(1) CM-Sepharose fast flow (Pharmacia) in 20 mM ammonium acetate, pH 5, and gradient elution between 0 and 1 M NaCl;

(2) Chelating Sepharose fast flow (Pharmacia) loaded with $Zn^{2+}$ and conditioned in 0,1 M Na-acetate and 1 M NaCl, pH 4, equilibrated with phosphate-buffered saline (10 mM Na-phosphate, pH 7,4, 0,15 M NaCl), and eluted with a gradient between phosphate buffered saline plus 1 M NaCl and 0,1 M Na-acetate, ph 4 plus 1 M NaCl;

(3) HPLC on Vydac C-4 in 0,1% trifluoroacetic acid, and eluted with 30 - 50% acetonitril in 0,1% trifluoroacetic acid;

(4) Sephacryl 200 (Pharmacia) in phosphate-buffered saline. After exhaustive dialysis against water the purified protein was freeze-dried and stored at 20°C.

The IL-4 variants were expressed in E. coli, renatured and purified to homogeneity as described (Kruse et al., 1993). The cDNA of IL-4 was modified by cassette mutagenesis, integrating synthetic double-stranded DNA with the desired mutation between engineered single restriction sites of wild type IL-4 cDNA (Wang et al., 1997). The variant IL-4/cys had the C-terminal extension GPLECH.

Protein concentrations were determined by measuring the absorbancy at 278 nm, using an extinction coefficient $e_{278}$ = 8 860 $M^{-1}$ $cm^{-1}$ for IL-4, and $e_{278}$ = 61 795 $M^{-1}$ $cm^{-1}$ for gamex (Pace et al., 1995).

## Biosensor experiments

All experiments were carried out on a BIA2000 system (Pharmacia Biosensor) at 25°C at a flow rate of 10 µl $min^{-1}$ in HBS running buffer (10 mM Hepes, pH 7.4/150 mM NaCl/3.4 mM EDTA/0.005% surfactant P20) with a data collection rate of 2,5 $s^{-1}$.

Two different protocolls were applied for attaching IL4-BP, respectively IL-4 to the bioosensor matrix:

(1) A CM5 biosensor chip was first loaded with streptavidin in flow cells 1 and 2. Subsequently biotinylated [C182A, Q207C]IL4-BP was immobilized at the streptavidin matrix (Shen et al., 1996) of flow cell 2 at a density of 50 to 1500 resonance units (RU).

(2) The variant IL-4/cys was immobilized in flow cell 2 by the thiol-disulfide exchange coupling procedure (ligand thiol method) at a density of 150 to 300 RU as detailed in the BIApplications handbook (Pharmacia Biosensor AB, 1994).

Throughout the experiments employing immobilized IL4-BP, the following reaction cycle was applied using the commands COINJECT and QUICKINJECT and a sequential perfusion of flow cells 1 and 2:

(1) Perfusion with 0,2 µM IL-4 or IL-4 variant for 2 min;

(2) Perfusion with 0,2 µM IL-4 or IL-4 variant plus gamex at the indicated concentrations for 2 min;

(3) Perfusion with buffer alone for 5 min;

(4) Regeneration of free immobilized IL4-BP by a wash with 0,1 M acetic acid plus 1 M NaCl for 30 s.

The differential sensogram between the recordings from flow cells 2 (plus IL4-BP) and flow cell 1 (minus IL4-BP) was generated and evaluated for the kinetic and equilibrium constants (BIAevaluation 2.1 software). The constants for the rate of association $k_{on}$ were calculated by the A + B = AB model and the type 2 or 3 fitting routine using the time period of the association phase where ln dRU/dt was linear. The dissociation rate constants $k_{off}$ were evaluated from the first half-life-time of the dissociation phase where the decay was exponential. The small dissociation of IL-4 protein during that period was subtracted. The dissociation constant $K_d$ was evaluated from the concentration dependence of the equilibrium binding, or calculated $K_d = k_{off}/k_{on}$ . The mean standard error was 17% +/- 10% for the $k_{on}$ values and 21% +/-13% for the $k_{off}$ values.

Two types of experiments were performed with immobilized IL-4:

Ternary complex with gamex
A protocoll similar to that described above for immobilized IL4-BP was applied but 0,5µM IL4-BP was added during steps 1 and 2 instead of IL-4.

Binary complex with gamex
The following reaction cycle was applied using the commands KINJECT and QUICKINJECT:

(1) Perfusion with the indicated concentrations of gamex for 2 min;
(2) Perfusion with buffer alone for 5 min;
(3) Regeneration of unliganded immobilized IL-4 by a wash with 0,1 M acetic acid plus 1 M NaCl for 30 s.

T-cell proliferation was measured by [3-H]thymidine incorporation into PHA blasts after a 3-day incubation with IL-4 or IL-4 variants at 12 2-fold dilutions (Kruse et al., 1992). Inhibition of IL-4 dependent T-cell proliferation was determined in the presence of 2 nM IL-4 and 12 2-fold dilutions of potential antagonists. The data were fitted by means of the Grafit (Erithacus software)program.

**References**

Aman, M. J., Tayebi, N., Obiri, N. I., Puri, R. K., Modi, W. S. and Leonard, W. J. (1996) J Biol Chem, 271, 29265-29270.

Bamborough, P., Hedgecock, C. J. and Richards, W. G. (1994) Structure, 2, 839-851.

Cunningham, B. C. and Wells, J. A. (1993) J Mol Biol, 234, 554-563.

Darnell, J. E. Jr, Kerr, I. M. and Stark, G. R. (1994) Science, 264, 1415-1421.

Duschl, A. (1995) Eur J Biochem, 228, 305-310.

Duschl, A. and Sebald, W. (1996) Eur Cytokine Netw, 7, 37-49.

Garcia, K. C., Scott, C. A., Brunmark, A., Carbone, F. R., Peterson, P. A., Wilson, I. A. and Teyton, L. (1996) Nature, 384, 577-581.

Gauchat, J. F., Schlagenhauf, E., Feng, N. P., Moser, R., Yamage, M., Jeannin, P., Alouani, S., Elson, G., Notarangelo, L. D., Wells, T., Eugster, H. P. and Bonnefoy, J. Y. (1997) Eur. J. Immunol., 27, xx-zz.

Heldin, C. H. (1995) Cell, 80, 213-223.

Hoffman, R. C., Castner, B. J., Gerhart, M., Gibson, M. G., Rasmussen, B. D., March, C. J., Weatherbee, J., Tsang, M., Gustchina, A., Schalk Hihi, C. et al. (1997) Protein Sci, 6, 494.

Ihle, J. N. (1995) Nature, 377, 591-594.

Kruse, N., Tony, H. P. and Sebald, W. (1992) EMBO J, 11, 3237-3244.

Kruse, N., Shen, B. J., Arnold, S., Tony, H. P., Muller, T. and Sebald, W. (1993) EMBO J, 12, 5121-5129.

Leonard, W. J. (1994) Curr Opin Immunol, 6, 631-635.

Miloux, B., Laurent, P., Bonnin, O., Lupker, J., Caput, D., Vita, N. and Ferrara, P. (1996) FEBS Lett-, 401, 163-166.

Müller, T., Sebald, W. and Oschkinat, H. (1994) Nat Struct Biol, 1, 674-676.

Müller, T., Dieckmann, T., Sebald, W. and Oschkinat, H. (1994a) J Mol Biol, 237, 423-436.

Rozwarski, D. A., Gronenborn, A. M., Clore, G. M., Bazan, J. F., Bohm, A., Wlodawer, A., Hatada, M. and Karplus, P. A. (1994) Structure, 2, 159-173.

Schnarr, B., Ezernieks, J., Sebald, W. and Duschl, A. (1997) Int Immunol, 9, 101-108.

Shen, B. J., Hage, T. and Sebald, W. (1996) Eur J Biochem, 240, 252-261.

Sugamura, K., Asao, H., Kondo, M., Tanaka, N., Ishii, N., Nakamura, M. and Takeshita, T. (1995) Adv Immunol, 59, 225-227.

Sugamura, K., Asao, H., Kondo, M., Tanaka, N., Ishii, N., Ohbo, K., Nakamura, M. and Takeshita T. (1996) Annu Rev Immunol, 14, 179-205.

Takeshita, T., Asao, H., Ohtani, K., Ishii, N., Kumaki, S., Tanaka, N., Munakata, H., Nakamura, M. and Sugamura, K. (1992) Science, 257, 379-382.

Tony, H. P., Shen, B. J., Reusch, P. and Sebald, W. (1994) Eur J Biochem, 225, 659-665.

Valitutti, S., Muller, S., Cella, M., Padovan, E. and Lanzavecchia, A. (1995) Nature, 375, 148-151.

Wang, Y., Shen, B. J. and Sebald, W. (1997) Proc. Natl. Acad. Sci. USA, 94, 1657-1662.

Wells, J. A. (1994) Curr Opin Cell Biol, 6, 163-173.

Wells, J. A. (1996) Proc Natl Acad Sci U S A, 93, 1-6.

Table 1

| Rate constants for the association ($k_{on}$) and dissociation ($k_{off}$) of gamex and a 1:1 complex between IL4-BP and IL-4 alanine variants. Sensograms were evaluated as detailed under Methods. ND means that a sensogram could not be evaluated due to low gamex binding. | | | | | |
|---|---|---|---|---|---|
| Alanine variants | $k_{on}$ x $10^{-4}$ $M^{-1}s^{-1}$ | $k_{on}$(mut)/$k_{on}$(IL4) | $k_{off}$ x 10 $s^{-1}$ | $k_{off}$(mut)/$k_{off}$(IL-4) | $K_d$($k_{off}$ / $k_{on}$) μM | $K_d$(mut)/$K_d$(IL-4) |
| I5A | 3,8 | 1,2 | 1,2 | 1,2 | 3,2 | 0,9 |
| T6A | 3,3 | 1,0 | 1,00 | 0,9 | 3,1 | 0,9 |
| L7A | 2,3 | 0,7 | >5 | >4,7 | >22 | >7 |
| Q8A | 2,9 | 0,9 | 1,2 | 1,2 | 4,2 | 1,2 |
| I11A | 2,1 | 0,7 | >8 | >7,1 | >36 | >11 |
| K12S | 1,7 | 0,5 | 1,5 | 1,4 | 8,9 | 2,6 |

Table 1 (continued)

| Rate constants for the association ($k_{on}$) and dissociation ($k_{off}$) of gamex and a 1:1 complex between IL4-BP and IL-4 alanine variants. Sensograms were evaluated as detailed under Methods. ND means that a sensogram could not be evaluated due to low gamex binding. | | | | | | |
|---|---|---|---|---|---|---|
| Alanine variants | $k_{on} \times 10^{-4}$ M⁻¹s⁻¹ | $k_{on}$(mut)/$k_{on}$(IL4) | $k_{off} \times 10$ s⁻¹ | $k_{off}$(mut)/$k_{off}$(IL-4) | $K_d$($k_{off}$/$k_{on}$) µM | $K_d$(mut)/$K_d$(IL-4) |
| T13A | 2,9 | 0,9 | 1,0 | 1,0 | 3,5 | 1,0 |
| N15A | 2,6 | 0,8 | 4,5 | 4,2 | 17 | 5,0 |
| S16A | 3,5 | 1,1 | 1,1 | 1,1 | 3,2 | 0,9 |
| E19A | 3,1 | 1,0 | 0,9 | 0,9 | 3 | 0,9 |
| E114A | 2,1 | 0,7 | 1,7 | 1,6 | 7,9 | 2,3 |
| R115A | 3,0 | 0,9 | 1,0 | 1,0 | 3,5 | 1,0 |
| K117A | 2,8 | 0,9 | 1,7 | 1,6 | 6 | 1,8 |
| T118A | 2,7 | 0,9 | 1,9 | 1,8 | 6,9 | 2,0 |
| R121A | 3,5 | 1,1 | 3,5 | 3,3 | 9,9 | 2,9 |
| E122A | 3,1 | 1,0 | 2,5 | 2,4 | 8 | 2,4 |
| K123A | ND | | ND | | ND | ND |
| Y124A | ND | | >10 | | ND | ND |
| S125A | 2,8 | 0,9 | 2,3 | 2,2 | 8,2 | 2,4 |
| IL-4 | 3,2 | 1,0 | 1,1 | 1,0 | 3,4 | 1,0 |

Table 2

| IL-4 variants are more severely affected in gamex-binding than in biological activity. The dissociations constants $K_d$ were evaluated from equilibrium binding of IL-4 variants to immobilized IL4-BP. The maximal response Rmax elicited by IL-4 variants during T-cell proliferation was related to the maximal response produced by IL-4. The $EC_{50}$ values were 120 +/- 35 pM for all variants. | | | | | | | |
|---|---|---|---|---|---|---|---|
| | Equilibrium binding | | T-cell proliferation | | Equilibrium binding | | T-cell proliferation |
| Alanine variants | $K_d$ µM | $K_d$(mut)/$K_d$(IL-4) | Rmax(IL-4)/Rmax(var) | Charge variants | $K_d$ µM | $K_d$(mut)/$K_d$(IL-4) | Rmax(IL-4)/Rmax(var) |
| 15A | 4,1 | 1,5 | | | | | |
| T6A | 3,3 | 1,2 | | | | | |
| L7A | 31 | 11 | 1,3 | L7D | 3,3 | 1,2 | 1,0 |
| Q8A | 4,1 | 1,5 | | Q8R | 13 | 4,0 | 0,9 |
| I11A | 79 | 28 | 1,5 | I11D | >300 | >100 | 6,0 |
| K12S | 7,1 | 2,5 | | | | | |
| T13A | 3,1 | 1,1 | | | | | |
| N15A | 15 | 5,4 | 1,2 | N15D | 70 | 25 | 1,2 |
| S16A | 2,9 | 1,0 | | | | | |
| E19A | 2,2 | 0,8 | | | | | |
| E114A | 11 | 3,8 | 1,1 | E114R | 12 | 4,0 | 1,2 |

Table 2 (continued)

| IL-4 variants are more severely affected in gamex-binding than in biological activity. The dissociations constants $K_d$ were evaluated from equilibrium binding of IL-4 variants to immobilized IL4-BP. The maximal response Rmax elicited by IL-4 variants during T-cell proliferation was related to the maximal response produced by IL-4. The $EC_{50}$ values were 120 +/- 35 pM for all variants. | | | | | | | |
|---|---|---|---|---|---|---|---|
| | Equilibrium binding | | T-cell proliferation | | Equilibrium binding | | T-cell proliferation |
| Alanine variants | $K_d$ µM | $K_d$(mut)/$K_d$(IL-4) | Rmax(IL-4)/Rmax(var) | Charge variants | $K_d$ µM | $K_d$(mut)/$K_d$(IL-4) | Rmax(IL-4)/Rmax(var) |
| R115A | 2,5 | 0,9 | | R115D | 1,5 | 0,5 | |
| K117A | 5,6 | 2,0 | 1,1 | K117D | 4,0 | 1,4 | |
| T118A | 8 | 2,9 | 1,1 | T118K | 100 | 36 | 1,4 |
| R121A | 9,3 | 3,3 | 1,1 | R121D | >300 | >100 | 5,3 |
| E122A | 9,2 | 3,3 | | E122R | 11 | 3,9 | 1,5 |
| K123A | >300 | >100 | 6,3 | K123D | 7,0 | 2,5 | 1,4 |
| Y124A | 220 | 78 | 2,0 | Y124D | >300 | >100 | >100 |
| S125A | 7,7 | 2,8 | 1,0 | S125D | >300 | >100 | 5,4 |
| IL-4 | 2,8 | 1,0 | 1,0 | | | | |

## Claims

1. hIL-4 muteins having a reduced affinity and/or an altered specificity to the γ subunit of the IL-4 receptor and/or HIL-13 R α subunit of the hIL-4 receptor.

2. The hIL-4 mutein according to claim 1 having a replacement of a natural occurring aminoacid in the A-helix of hIL-4 by another aminoacid.

3. The hIL-4 mutein according to claim 2 having a replacement in one or more of positions 11, 15, 12 and 7.

4. hIL-4 muteins according to any of claims 1 to 3 having additional replacement in one or more of positions 121, 123, 124 and 125.

5. A DNA coding for a mutein according to anyone of the claims 1 to 4.

6. A vector comprising the DNA according to claim 5.

7. A microorganism comprising the vector according to claim 6.

8. A method of making a mutein according to anyone of claim 1 to 4, wherein the microorganism of claim 7 is cultivated and the mutein is isolated from the culture.

**European Patent Office**

## EUROPEAN SEARCH REPORT

Application Number

EP 97 11 8219

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | Y. WANG ET AL: "A mixed-charge pair in human interleukin-4 dominates high-affinity interaction with the receptor alpha chain" PROCEEDINGS OF THE NATIONAL ACADEMY OF SCIENCES OF USA., vol. 94, March 1997, WASHINGTON US, pages 1657-1662, XP002061699 * page 1658; table 1 * * page 1661; table 2 * | 1-3,5-8 | C12N15/24 C12N1/21 C07K14/54 |
| X | D.J. MATTHEWS ET AL: "X-SCID B cell responses to interleukin-4 and interleukin-13 are mediated by a receptor complex that includes the interleukin-4 receptor alpha chain(p140) but not the gamma c chain" EUROPEAN JOURNAL OF IMMUNOLOGY, vol. 27, January 1997, pages 116-121, XP002061700 * abstract * * page 120, left-hand column * | 1 | |
| D,X | KRUSE N ET AL: "TWO DISTINCT FUNCTIONAL SITES OF HUMAN INTERLEUKIN 4 ARE IDENTIFIED BY VARIANTS IMPAIRED IN EITHER RECEPTOR BINDING OR RECEPTOR ACTIVATION" EMBO JOURNAL, vol. 12, no. 13, 15 December 1993, pages 5121-5129, XP000565734 * page 5123, right-hand column * * page 5125 * | 1,2 | **TECHNICAL FIELDS SEARCHED (Int.Cl.6)** C07K C12N |

-/--

The present search report has been drawn up for all claims

| Place of search | Date of completion of the search | Examiner |
|---|---|---|
| THE HAGUE | 7 April 1998 | Le Cornec, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

European Patent
Office

**Application Number**

EP 97 11 8219

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | L. RAMANATHAN ET AL: "Immunochemical mapping of domains in human interleukin-4 recognized by neutralizing monoclonal antibodies" BIOCHEMISTRY., vol. 32, 1993, EASTON, PA US, pages 3549-3556, XP002061701 * page 3554; table IV * | 1,5-8 | |
| D,A | A. DUSCHL ET AL: "Transmembrane and intracellular signalling by interleukin-4 . Receptor dimerization and beyond" EUROPEAN JOURNAL OF CYTOKINE NETWORK, vol. 7, no. 1, January 1996 - March 1996, pages 37-49, XP002061702 | | |
| A,D | A. DUCSHL : "An antagonistic mutant of interleukin-4 fails to recruit gamma c into the receptor complex" EUROPEAN JOURNAL OF BIOCHEMISTRY , vol. 228, no. 2, March 1995, pages 305-310, XP002061703 * the whole document * | 1-8 | **TECHNICAL FIELDS SEARCHED** (Int.Cl.6) |

The present search report has been drawn up for all claims

| Place of search | Date of completion of the search | Examiner |
|---|---|---|
| THE HAGUE | 7 April 1998 | Le Cornec, N |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...............................................................
& : member of the same patent family, corresponding
document